# EUROPEAN PATENT SPECIFICATION

(11) **EP 3 417 286 B1**
(45) Date of publication and mention of the grant of the patent: **22.09.2021**
(21) Application number: 17718439.7
(22) Date of filing: 14.03.2017
(51) Int. Cl.: G01N 33/36, G01L 5/00, G01N 33/00

(54) **A NOVEL PRESSURE MEASURING TEST APPARATUS FOR CONVENTIONAL AND TECHNICAL TEXTILE PRODUCTS**
NEUARTIGE DRUCKMESSENDE TESTVORRICHTUNG FÜR KONVENTIONELLE UND TECHNISCHE TEXTILPRODUKTE
NOUVEL APPAREIL D'ESSAI DE MESURE DE PRESSION POUR PRODUITS TEXTILES CONVENTIONNELS ET TECHNIQUES

(30) Priority: 17.03.2016 TR 201603486
(43) Date of publication of application: 26.12.2018
(73) Proprietor: Calik Denim Tekstil Sanayi Ve Ticaret Anonim Sirketi, Güngören/Istanbul (TR); Babaarslan, Osman, 01330 Balcali Saricam Adana (TR); Yilmaz, Recep, Melikgazi/Kayseri (TR); Seyrek Kurban, Nazime, Balcali Saricam/Adana (TR)
(72) Inventor: BABAARSLAN, Osman, Balcali Saricam/Adana (TR); YILMAZ, Recep, Balcali Saricam/Adana (TR); SEYREK KURBAN, Nazime, Balcali Saricam/Adana (TR)
(74) Representative: Yamankaradeniz, Kemal
(86) International application number: PCT/TR2017/050099
(87) International publication number: WO 2017/160255

(56) References cited:
- EP-A2- 1 118 851
- YONGRONG WANG ET AL: "A smart mannequin system for the pressure performance evaluation of compression garments", TEXTILE RESEARCH JOURNAL, vol. 81, no. 11, 23 March 2011 (2011-03-23), pages 1113-1123, XP055375059, GB ISSN: 0040-5175, DOI: 10.1177/0040517511398942

## Description

### Technical Field

The invention relates to test apparatuses which allow measuring and evaluating the pressure values to be applied by textile products, especially lower body clothing, to certain parts of the human body.

The invention particularly relates to the test apparatuses which are used for pressure measurement evaluation to be applied by textile products, especially lower body clothing, to the body; which permit interpreting sanitary and comfort properties of the products by means of digital data before being presented to the end user; the dimensions of which can be changed; and which, by mimicking body movements, are provided with dynamic performance.

### State of the Art

In clothing industry today, various technological innovations are being made in order that the users will wear textile products in a comfortable and healthy manner. What is aimed by these innovations is to perform manufacturing in accordance with the pressure value suitable for the comfort and sanitary requirements and standards (whether a decreasing pressure from the ankle to the crotch is present, whether excessive pressure is present in the respective region, etc. for ensuring a proper blood flow) of the user by determining the pressure applied by the textile products, especially by the lower body clothing, e.g. socks, trousers, shorts, etc., while they are on the user. It is particularly of importance to adjust manufacturing by conducting said evaluations in medical technical textile products such compression stockings and support stockings, which are used for treating some diseases.

In the state of the art, various test apparatuses have been developed and innovations have been made for the measurement and standardization of said pressure values. Some of these include individual leg molds made of wood according to different stocking sizes. The circumference and length of the leg is adjusted by means of these molds. Test probes are attached to these wood molds and the values from the probes are measured by means of a control system. In such devices, pressure measurement is performed by a manometer with digital output operated by pneumatic principle, some of which allow pressure measurement from seven different points. At the same time, the measurement is conducted in a static (stationary) test environment. In this case, the pressure values which occur during different movements of the leg cannot be measured. Such devices have been developed for particular use in the pressure measurement of medical stockings.

The Patent Application No. US20070012120A1 discloses a mechanical leg developed for allowing size change in a different test apparatus used for pressure measurement of medical stockings. In this test apparatus, circumferential size expansion is ensured electronically and unidirectionally. Since the leg allows for a unidirectional expansion in this apparatus, a proper measurement can only be made in a limited region.

The search on the state of the art revealed some other documents. The Patent Application No. TR2010/01394 relates to a pressure measurement apparatus. It is stated in said invention that pressure measurement at seven different points of the leg can be performed in different types (short, mid-calf, thigh high, and pantyhose) and various groups (primarily compression stockings; as well as regular, support, and work stockings) of stockings and the pressure profile can be detected prior to wearing. It was also stated that this apparatus is capable of determining pressure values of cuff portion of regular socks, at the same time allowing a pressure measurement for any type of compression product (bandages, corsets, knee pads, wrist pads, etc.) by adjusting the mechanical mold form. However, this apparatus also performs a static measurement and it is not possible hereby to detect instantaneous measurement values against tensions. Moreover, this test apparatus is quite different from human anatomy and the surface structure thereof carries many components that may affect the test result, e.g. nuts, plates; as a consequence, a correct measurement is not possible.

An European patent application with the publication number of EP1118851 A2 relates a device for measuring the compression that can be exerted by a stocking, a pantyhose or the like. The device disclosed in the document is capable of displaying and recording the compression data both in a static and in a dynamic condition, uses a detection system with two-dimensional sensors (3), each comprising a matrix arrangement of load cells.

It appears that the pressure measurement apparatuses worldwide are generally statically operating apparatuses. The moving apparatuses, on the other hand, have a limited capability of movement, but not a sufficient measurement precision.

As a result, the need for test apparatuses which are used for pressure measurement evaluation of textile products, especially lower body clothing; which permit interpreting sanitary and comfort properties of textile products before being presented to the end user; the dimensions of which can be changed; and which, by mimicking body movements, are provided with dynamic performance, and the insufficiency of the existing solutions have made it obligatory to make a development in the related technical field.

### Objects of the Invention

The present invention relates to a pressure measuring test apparatus capable of performing dynamic and static measurement, which meets the aforementioned requirements, eliminates all the drawbacks, and at the same time provides additional advantages.

The primary object of the dynamic and static pressure measuring test apparatus according to the invention is to provide a model having sensors thereon in order to be able to define the pressure values, as well as the pressure difference, to be applied by textile products, especially lower body clothing, to the human body and body parts while they are on. Thanks to being able to change the length (height) and width (diameter) measures of this model instantaneously by way of driving elements, it is possible to measure pressure changes and pressure values occurring at different body positions in different product sizes (S, M, L XL, etc.) by means of the same test apparatus. Thus, it will be possible to evaluate textile products at a wider range, and hence to evaluate their convenience in terms of clothing and produce ideas regarding the development thereof. Further, the ability to adjust the length and width allows for the applicability of the same textile product to different sizes and eliminates the requirement of additional components or machines thanks to a universal design. At the same time, instantaneous pressure values can be measured by stabilizing the apparatus at a position and the effects of repeated movements on the garment can be investigated.

Another object of the invention is to mimic the movements of the body and its parts by fastening the models to one another using moveable joints. Hence, pressure values to be applied by the textile product to the user at different positions can be obtained. The former characteristic allows for performing measurements in the moveable joints, e.g. wear, resilience measurements.

And another object of the invention is to ensure that said movement takes place automatically by means of the driving element which drives the joints. With the continuous movement of the driving element, the pressure values applied by the textile product to the user even when s/he is moving will be obtained.

In order to achieve the aforementioned objects, a pressure measuring test apparatus according to claim 1 is provided.

The structural and characteristic features and all advantages of the invention will be understood more clearly by referring to the following figures and the detailed description written with reference to these figures; therefore, these figures and the detailed description should be taken into consideration while making an evaluation.

### Figures for a Better Understanding of the Invention

The embodiment of the present invention and advantages thereof with the additional components must be considered together with the drawings explained below in order to be fully understood.
FIG. 1a: Front view of the pressure measuring test apparatus according to the invention.
FIG. 1b: Side view of the pressure measuring test apparatus according to the invention.
FIG. 2: Side view of the model in the form of a leg within the pressure measuring test apparatus according to the invention.
FIG. 3: Partial internal view of the model in the form of a leg within the pressure measuring test apparatus according to the invention.
FIG. 4: Side view of the model in the form of a leg within the pressure measuring test apparatus according to the invention in which the linear expansion movements of said leg are illustrated.
FIG. 5a, 5b: Side views of the model in the form of a leg within the pressure measuring test apparatus according to the invention at two different positions connected to the frame.
FIG. 6a, 6b, 6c: Side, front, and perspective views of the model in the form the entire body within the pressure measuring test apparatus according to the invention when in vertical position.
FIG. 7a, 7b, 7c: Side, front, and perspective views of the model in the form the entire body within the pressure measuring test apparatus according to the invention when in sitting position.
FIG. 8a, 8b, 8c: Side, front, and perspective views of the model in the form the entire body within the pressure measuring test apparatus according to the invention when in walking/knee bending position.

The drawings do not necessarily need to be scaled and the details that are not required for understanding the invention may have been omitted. Apart from that, the elements that are at least substantially identical or have at least substantially identical functions are referred with the same reference numeral.

### Part References

- 10: Pressure measuring test apparatus
- 11: Frame
- 12: Control system
- 13: Lifting means
- 20: Model
- 21: Sensor
- 22: Outer surface
- 22.1: Form pieces
- 23: Body
- 23.1: Joints
- 24: Length driving element
- 25: Diameter driving element
- 25.1: Fixation contact tips
- 26: Hinge
- 26.1: Upper hinge slot
- 26.2: Lower hinge slot
- 27: Hinge driving element
- 30: Stays
- A: Length movement
- B: Diameter movement
- C: Bending movement

### Detailed Description of the Invention

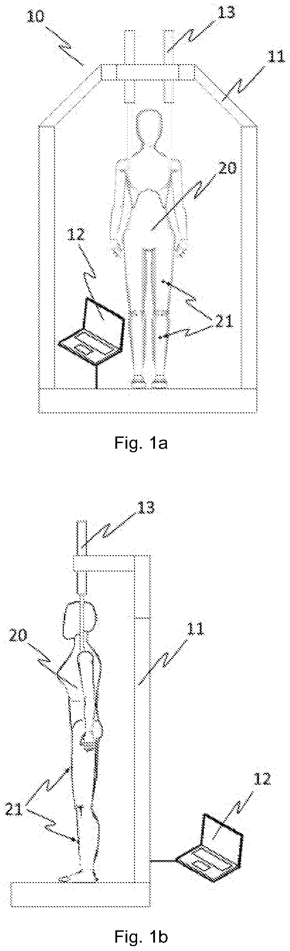

Figs. 1a and 1b show front and side views of the pressure measuring test apparatus (10) according to the invention, respectively. The model (20) to be dressed with the textile products whose pressure values will be measured is shown in entire body form. Nevertheless, only a part or some parts of the body may as well be used according to the intended use. Although the detailed description of the model (20) when adjusted in the leg embodiment is given in the present invention, said embodiment is applicable to all parts and organs of the body.

The dimensions of the pressure measuring test apparatus (10) can be changed and it may be provided with a dynamic performance by mimicking body movements thanks to the sensor (21) and control system (12) comprised therein, said apparatus being developed for use in pressure measurement evaluation of textile products, especially lower body clothing, and for interpreting sanitary and comfort properties of textile products before being presented to the end user. In this context, the overall configuration basically comprises a main body (23) consisting of a plurality of joints (23.1) performing linear movement in vertical z axis and bending movement in x axis and being engaged in telescopic manner. Also comprised are outer surfaces (22) which surround said body (23) and have a plurality of sensors (21) and a plurality of form pieces (22.1) thereon, as well as a plurality of pieces in the form of a human leg. Likewise, at least one length driving element (24) which provides the length movement (A) of the body (23) in vertical z axis and at least one diameter driving element (25) which provides the diameter movement (B) of the body (23) in horizontal y axis are comprised. The invention further comprises a hinge (26) having a hinge driving element (27) which provides the bending movement (C) of the body (23) in x axis and at least one model (20) to be dressed with the textile product whose pressure values are to be obtained. Moreover, the invention comprises a hinge driving element (27) which ensures the bending movement (C) in x axis and which is positioned on the hinge (26) in crosswise or vertical manner by means of the upper hinge slot (26.1) and the lower hinge slot (26.2).

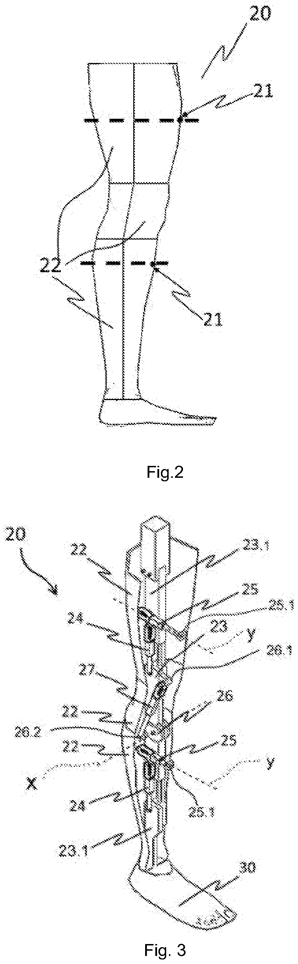

Fig. 2 shows the side view of the model (20) in the form of a leg within the pressure measuring test apparatus (10). As in this one and the other models (20), the outer surface (22) not only gives the outer form but also provides the basis for the textile product to be worn. The sensors (21) located on the outer surface (22) measures the amount of pressure and then transmits the same to the control system (12). The positions, numbers, and directions of the sensors (21) on the outer surface (22) may be defined according to the standards and customer requirements and adjusted as desired. The sensors (21) may be load-cell, FSR (Force Sensitive Resistor), or pneumatic sensors. The most important factor for mimicking the body parts anatomically is the outer surface (22). It is specially formed and may be one or more in number. The outer surface (22) is preferably made of rigid materials such that equivalent pressure values will be obtained thereon. However, it is possible to use flexible materials and the like for different embodiments and increase the functionality.

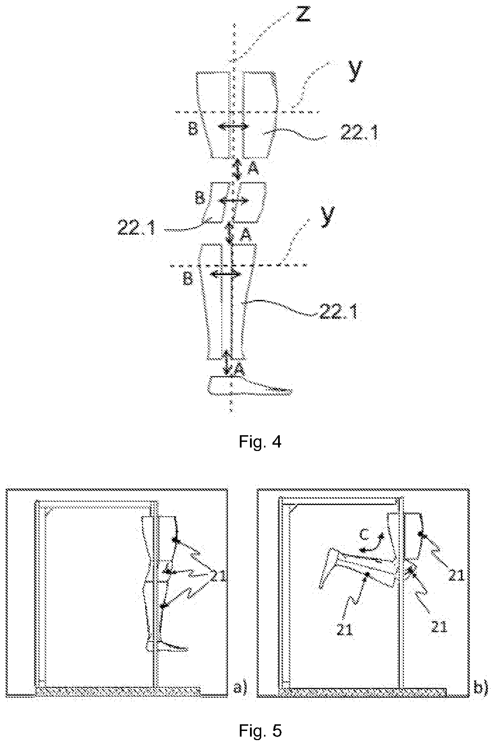

Fig. 3 shows the internal view of the model (20) in the form of a leg within the pressure measuring test apparatus (10). The body (23) forming the principal structure of the model (20) preferably consists of linear telescopic pieces. In such case, the length thereof can be changed. The outer surfaces (22) are disposed around the body (23). The length driving element (24) engaged with the pieces of the body (23) adjusts the length of the body (23) by driving the same. Similarly, the diameter driving element (25) connected to the outer surfaces (22) changes the distance between the outer surfaces (22) by driving the same. Thanks to these two properties, the dimensional properties of the model (20) can be changed, thereby increasing the applicability thereof for different body sizes and positions. The stays (30) is designed entirely in form. It may, however, be equipped with sensors (21) and configured as a model (20). In Fig. 4, the length movement (A) and diameter movement (B) of the models (20) in the form of a leg are indicated with linear movements. The fixation contact tips (25.1), which are secured on said outer surfaces (22) through the internal wall surface, are illustrated.

The models (20) may be stably interconnected, or alternatively they may be interconnected by means of a hinge (26), without requiring a stable fixation, such that they will be able to perform bending movement. The hinges (26) ensure the movement of the models (20) with respect to one another, thereby allowing it to mimic body movements. Especially when they are surrounded by an outer surface (22), the joints of the body can be exactly mimicked. The hinge driving element (27) controls the movement of the two or more models (20) around the hinge (26). Figs. 5a and 5b show side views of the models (20) in the form of leg at two different positions when connected to the frame (11). The bending movement (C) occurs during bending of the knee.

In the preferred embodiment of the invention, the length driving element (24), the diameter driving element (25), and the hinge driving elements (27) are all pneumatic, hydraulic, or electrical. Thus, less space is occupied and advantages in terms of design are provided.

The frame (11) used within the system ensures the structural positions of the model (20) or models (20). The lifting means (13), on the other hand, lifts the model (20) or models (20) having been formed, ensuring that they remain in the air in a contactless manner. The control system (12), which preferably is a computer, a PLC-, or PIC-based computer, compiles and records the data from the sensors (21). In addition, the control system (12) controls the actions of the length driving element (24), the diameter driving element (25), and the hinge driving elements (27).

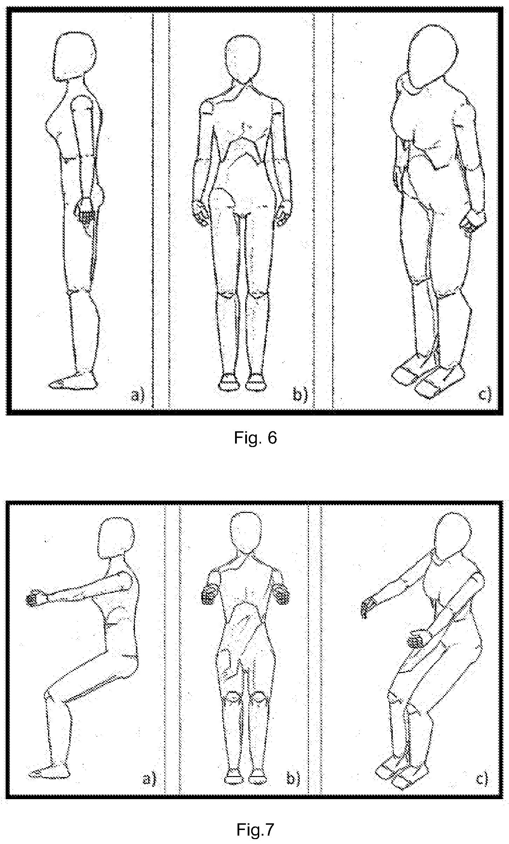

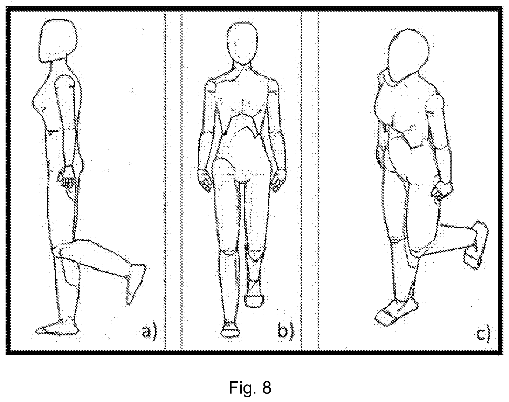

The pressure measuring test apparatus (10) allowing dynamic and static measurement which has been developed herein may, as emphasized above, cover the entire human body, or alternatively it may be reduced down to a specific design in accordance with the targeted test structure. What is meant here by the concept or "reducing" is that the model (20) may consist of a single leg, or it may be provided as a design consisting of an ankle. Figs. 6, 7, and 8 show the views of the model (20) from different perspectives, i.e. in standing (vertical), sitting, and walking positions, respectively. According to the targeted test structure, the model (20) or models (20) comprised in the pressure measuring test apparatus may be provided with the ability of movement such that they will perform different movement(s). For example, in case it is desired to test the behavior of the product to be tested during sitting/crouching movement, the model (20) structure in Fig. 6 is repeatedly taken to the position shown in Fig. 7. As another example, in case the behaviors during walking and/or running are to be analyzed in the product to be tested, the pressure measuring test apparatus (10) with movement amplitude as illustrated in Fig. 8 is provided. All movement capabilities to be incorporated in the models, including dimensional changes according to size, are directly ensured by mechanism technique discipline and by the length driving element (24), the diameter driving element (25), and the hinge driving elements (27).

With the pressure measuring test apparatus (10) according to the invention, it is possible to perform dynamic measurements as well as static measurements without any movement.

Working: The pre-test dimensional changes according to size on the models (20), which are fixed on the frame (11), are adjusted by the program operating on the control system (12) interface. Once the adjustment value is confirmed, the dimensional changes according to size start in the models (20). First, the lifting means (13) lifts the models (20) and eliminates the contact thereof with the ground. Afterwards, in line with the incoming data, the order may be changed and the outer surfaces (22) on the models (20) move away from one another and thus create a diameter movement (B). By the diameter movement (B), the required circumferential and dimensional upsizing-downsizing is performed according to the size type (S, M, L, etc.) of the textile product being tested. The stays (30) structure plays no role in dimensional changes according to size and allows the contact of the models (20) with the ground when necessary. The positions of the sensors (21) are predetermined and adjusted according to the standards and customer requirements. An increase in the number of sensors (21) is advantageous in terms of the demanded result resolution. The order may change in line with the commands coming from the control system (12) software and length movements (A) are performed on the bodies (23). Later, the model (20) is dressed with the textile product to be tested and fixed. The initiation command is given over the control system (12). In accordance with the demanded test pattern, the pressure measuring test apparatus (10) performs one or several of fabric puncture, walking, and crouching/sitting movements and records the variable data coming from the sensors (21) throughout the process. For instance, when observing the knee bending movement, is will be seen that hinge driving element (27) in the knee region bends the hinge (26) at the level of the kneecap at a predetermined angle value, thereby creating the position. While the movements are being completed, the recorded test pressure values are collected by the control system (12). Upon completion of the test, the pressure measuring test apparatus (10) assumes its original position and it is removed from the test sample. The sensors (21) are calibrated in idle position and thus the pressure measuring test apparatus (10) is reset and becomes ready for the next test procedure.

## Claims

1. A pressure measuring test apparatus (10) suitable for measuring pressure of textile products to the human body comprising:
a plurality of sensors (21) adapted to detect the pressure values applied by textile products,
a control system (12) adapted to receive the information from said sensors (21),
**characterized in that** it comprises:
- a main body (23) comprising a plurality of joints (23.1) adapted to mimic human movements, and adapted to change the dimensions of the main body (23) wherein the main body (23) is adapted to change its length along the vertical direction (z- axis), the horizontal direction (y axis) and the bending direction (x axis),
- outer surfaces (22) surrounding said main body (23) and having the plurality of sensors (21) and a plurality of form pieces (22.1) thereon,
- at least one length driving element (24) providing the length movement (A) of said main body (23) in vertical z axis,
- at least one diameter driving element (25) adapted to increase or decrease the diameter of said body (23) in the horizontal y axis (B),
- a hinge (26) having a hinge driving element (27) adapted to provide the bending movement (C) of said main body (23) in x axis, and
- at least one model (20) representing the human body or parts of the human body and comprising the main body (23) and adapted to be dressed with the textile product whose pressure values are to be obtained.

2. The pressure measuring test apparatus (10) according to Claim 1, **characterized in that** the main body (23) comprises a plurality of pieces engaged in telescopic manner and configured in vertical z axis.

3. The pressure measuring test apparatus (10) according to Claim 1, **characterized in that** the outer surfaces (23) comprise a plurality of pieces in the form of a human leg surrounding said body (23).

4. The pressure measuring test apparatus (10) according to Claim 1, **characterized in** comprising a hinge driving element (27) adapted to ensure the bending movement (C) in x axis and which is positioned on the hinge (26) by means of an upper hinge slot (26.1) and a lower hinge slot (26.2).

5. The pressure measuring test apparatus (10) according to Claim 1, **characterized in** comprising a hinge driving element (27) which is positioned in crosswise manner with respect to vertical z axis.

6. The pressure measuring test apparatus (10) according to Claim 1, **characterized in** comprising a length driving element (24), a diameter driving element (25), and hinge driving elements (27), said elements being pneumatic, hydraulic, or electrically driven.

7. The pressure measuring test apparatus (10) according to Claim 1, **characterized in** comprising a plurality of diameter driving elements (25) having fixation contact tips (25.1) which are secured on said outer surfaces (22) through the internal wall surface.

8. The pressure measuring test apparatus (10) according to Claim 1, **characterized in** comprising at least one frame (11) ensuring the structural positions of the model (20) or models (20).

9. The pressure measuring test apparatus (10) according to Claim 1, **characterized in** comprising at least one lifting means (13) for lifting the model (20) or models (20) and enabling them to remain in the air without contact with the ground.

10. The pressure measuring test apparatus (10) according to Claim 1, **characterized in that** the control system (12) is configured to compile and record the data from said sensors (21).

11. The pressure measuring test apparatus (10) according to Claim 1, **characterized in that** the control system (12) is configured to control the actions of said length driving element (24), diameter driving element (25), and hinge driving elements (27).

12. The pressure measuring test apparatus (10) according to Claim 1, **characterized in that** said pressure measuring test apparatus (10) is capable of static and/or dynamic measurement.

13. The pressure measuring test apparatus (10) according to Claim 1, **characterized in that** said sensors (21) are load-cell, FSR, or pneumatic sensors.

## Patentansprüche

1. Druckmess-Prüfvorrichtung (10), die zum Messen eines Drucks von Textilprodukten auf den menschlichen Körper geeignet ist, umfassend:
eine Vielzahl von Sensoren (21), die angepasst sind, um Druckwerte zu erfassen, die von den Textilprodukten ausgeübt werden,
ein Steuersystem (12), das angepasst ist, um die Informationen von den Sensoren (21) zu empfangen, **dadurch gekennzeichnet, dass** sie Folgendes umfasst:
- einen Hauptkörper (23), umfassend eine Vielzahl von Gelenken (23.1), die angepasst sind, um menschliche Bewegungen nachahmen, und angepasst sind, um die Abmessungen des Hauptkörpers (23) zu ändern, wobei der Hauptkörper (23) angepasst ist, um seine Länge entlang der vertikalen Richtung (z-Achse), der horizontalen Richtung (y-Achse) und der Biegerichtung (x-Achse) zu ändern,
- Außenflächen (22), die den Hauptkörper (23) umgeben und die Vielzahl von Sensoren (21) und eine Vielzahl von Formteilen (22.1) darauf aufweisen,
- mindestens ein Längenantriebselement (24), das die Längenbewegung (A) des Hauptkörpers (23) in vertikaler z-Achse bereitstellt,
- mindestens ein Durchmesserantriebselement (25), das geeignet ist, den Durchmesser des Körpers (23) in der horizontalen y-Achse (B) zu vergrößern oder zu verringern,
- ein Gelenk (26), das ein Gelenkantriebselement (27) aufweist, das angepasst ist, um die Biegebewegung (C) des Hauptkörpers (23) in x-Achse zu erzeugen, und
- mindestens ein Modell (20), das den menschlichen Körper oder Teile des menschlichen Körpers darstellt, und umfassend den Hauptkörper (23) und angepasst, um mit dem Textilprodukt bekleidet zu werden, dessen Druckwerte erlangt werden sollen.

2. Druckmess-Prüfvorrichtung (10) nach Anspruch 1, **dadurch gekennzeichnet, dass** der Hauptkörper (23) eine Vielzahl von teleskopartig ineinandergreifenden und in vertikaler z-Achse konfigurierten Teilen umfasst.

3. Druckmess-Prüfvorrichtung (10) nach Anspruch 1, **dadurch gekennzeichnet, dass** die Außenflächen (23) eine Vielzahl von Teilen in Form eines menschlichen Beins umfassen, die den Körper (23) umgeben.

4. Druckmess-Prüfvorrichtung (10) nach Anspruch 1, **dadurch gekennzeichnet, dass** sie ein Gelenkantriebselement (27) umfasst, das angepasst ist, um die Biegebewegung (C) in x-Achse zu gewährleisten und das mittels eines oberen Gelenkschlitzes (26.1) und eines unteren Gelenkschlitz (26.2) an dem Gelenk (26) positioniert ist.

5. Druckmess-Prüfvorrichtung (10) nach Anspruch 1, **dadurch gekennzeichnet, dass** sie ein Gelenkantriebselement (27) umfasst, das quer in Bezug auf die vertikale z-Achse positioniert ist.

6. Druckmess-Prüfvorrichtung (10) nach Anspruch 1, **dadurch gekennzeichnet, dass** sie ein Längenantriebselement (24), ein Durchmesserantriebselement (25) und Gelenkantriebselemente (27) umfasst, wobei die Elemente pneumatisch, hydraulisch oder elektrisch angetrieben werden.

7. Druckmess-Prüfvorrichtung (10) nach Anspruch 1, **dadurch gekennzeichnet, dass** sie eine Vielzahl von Durchmesserantriebselementen (25) umfasst, die Fixierkontaktspitzen (25.1) aufweisen, die durch die Innenwandfläche hindurch an den Außenflächen (22) befestigt sind.

8. Druckmess-Prüfvorrichtung (10) nach Anspruch 1, **dadurch gekennzeichnet, dass** sie mindestens einen Rahmen (11) umfasst, der die strukturellen Positionen des Modells (20) oder der Modelle (20) gewährleistet.

9. Druckmess-Prüfvorrichtung (10) nach Anspruch 1, **dadurch gekennzeichnet, dass** sie mindestens eine Hebeeinrichtung (13) zum Anheben des Modells (20) oder der Modelle (20) und zum Ermöglichen ihres Verbleibs in der Luft ohne Kontakt mit dem Boden umfasst.

10. Druckmess-Prüfvorrichtung (10) nach Anspruch 1, **dadurch gekennzeichnet dass** das Steuersystem (12) konfiguriert ist, um die Daten der Sensoren (21) zusammenzustellen und aufzuzeichnen.

11. Druckmess-Prüfvorrichtung (10) nach Anspruch 1, **dadurch gekennzeichnet dass** das Steuersystem (12) konfiguriert ist, um die Aktionen des Längenantriebselements (24), des Durchmesserantriebselements (25) und der Gelenkantriebselemente (27) zu steuern.

12. Druckmess-Prüfvorrichtung (10) nach Anspruch 1, **dadurch gekennzeichnet, dass** die Druckmess-Prüfvorrichtung (10) zur statischen und/oder dynamischen Messung in der Lage ist.

13. Druckmess-Prüfvorrichtung (10) nach Anspruch 1, **dadurch gekennzeichnet, dass** die Sensoren (21) Kraftmesszellen-, FSR- oder pneumatische Sensoren sind.

## Revendications

1. Appareil d'essai de mesure de pression (10) approprié pour mesurer la pression de produits textiles sur le corps humain, comprenant :
une pluralité de capteurs (21) adaptés à la détection des valeurs de pression appliquées par des produits textiles,
un système de commande (12) adapté à la réception des informations en provenance desdits capteurs (21),
**caractérisé en ce qu'**il comprend :
- un corps principal (23) comprenant une pluralité d'articulations (23.1) adaptées à l'imitation de mouvements humains et adaptées au changement des dimensions du corps principal (23), ledit corps principal (23) étant adapté au changement de sa longueur le long de la direction verticale (axe z), la direction horizontale (axe y) et la direction de flexion (axe x),
- des surfaces externes (22) entourant ledit corps principal (23) et comportant la pluralité de capteurs (21) et une pluralité de pièces de forme (22.1) sur elles,
- au moins un élément d'entraînement longitudinal (24) assurant le mouvement longitudinal (A) dudit corps principal (23) dans l'axe z vertical,
- au moins un élément d'entraînement diamétral (25) adapté à l'augmentation ou à la diminution du diamètre dudit corps (23) dans l'axe y horizontal (B),
- une charnière (26) comportant un élément d'entraînement de charnière (27) adapté à la production du mouvement de flexion (C) dudit corps principal (23) dans l'axe x, et
- au moins un modèle (20) représentant le corps humain ou des parties du corps humain et comprenant le corps principal (23) et conçu pour être habillé avec le produit textile dont on doit obtenir des valeurs de pression.

2. Appareil d'essai de mesure de pression (10) selon la revendication 1, **caractérisé en ce que** le corps principal (23) comprend une pluralité de pièces engagées de manière télescopique et configurées dans l'axe z vertical.

3. Appareil d'essai de mesure de pression (10) selon la revendication 1, **caractérisé en ce que** les surfaces externes (23) comprennent une pluralité de pièces sous la forme d'une jambe humaine entourant ledit corps (23).

4. Appareil d'essai de mesure de pression (10) selon la revendication 1, **caractérisé en ce qu'**il comprend un élément d'entraînement de charnière (27) adapté à la production du mouvement de flexion (C) dans l'axe x et qui est positionné sur la charnière (26) au moyen d'une fente de charnière supérieure (26.1) et d'une fente de charnière inférieure (26.2).

5. Appareil d'essai de mesure de pression (10) selon la revendication 1, **caractérisé en ce qu'**il comprend un élément d'entraînement de charnière (27) qui est positionné de manière croisée par rapport à l'axe z vertical.

6. Appareil d'essai de mesure de pression (10) selon la revendication 1, **caractérisé en ce qu'**il comprend un élément d'entraînement longitudinal (24), un élément d'entraînement diamétral (25) et des éléments d'entraînement de charnière (27), lesdits éléments étant à commande pneumatique, hydraulique ou électrique.

7. Appareil d'essai de mesure de pression (10) selon la revendication 1, **caractérisé en ce qu'**il comprend une pluralité d'éléments d'entraînement diamétral (25) comportant des pointes de contact de fixation (25.1) qui sont fixées sur lesdites surfaces externes (22) à travers la surface de paroi interne.

8. Appareil d'essai de mesure de pression (10) selon la revendication 1, **caractérisé en ce qu'**il comprend au moins un cadre (11) assurant les positions structurelles du modèle (20) ou des modèles (20).

9. Appareil d'essai de mesure de pression (10) selon la revendication 1, **caractérisé en ce qu'**il comprend au moins un moyen de levage (13) pour soulever le modèle (20) ou les modèles (20) et leur permettre de rester en l'air sans contact avec le sol.

10. Appareil d'essai de mesure de pression (10) selon la revendication 1, **caractérisé en ce que** le système de commande (12) est conçu pour compiler et enregistrer les données en provenance desdits capteurs (21).

11. Appareil d'essai de mesure de pression (10) selon la revendication 1, **caractérisé en ce que** le système de commande (12) est conçu pour commander les actions desdits élément d'entraînement longitudinal (24), élément d'entraînement diamétral (25) et éléments d'entraînement de charnière (27).

12. Appareil d'essai de mesure de pression (10) selon la revendication 1, **caractérisé en ce que** ledit appareil d'essai de mesure de pression (10) est capable d'effectuer une mesure statique et/ou dynamique.

13. Appareil d'essai de mesure de pression (10) selon la revendication 1, **caractérisé en ce que** lesdits capteurs (21) sont des capteurs de cellule de charge, FSR, ou pneumatiques.
